**Europäisches Patentamt**

**European Patent Office**

**Office européen des brevets**

(19)

(11) Veröffentlichungsnummer: **0 294 679 B1**

(12) # EUROPÄISCHE PATENTSCHRIFT

(45) Veröffentlichungstag der Patentschrift:
07.08.91 Patentblatt 91/32

(21) Anmeldenummer: **88108637.5**

(22) Anmeldetag: **30.05.88**

(51) Int. Cl.⁵: **A61G 7/10, A61B 6/04**

(54) Patientenlagerungsvorrichtung.

(30) Priorität: **12.06.87 DE 8708335 U**

(43) Veröffentlichungstag der Anmeldung:
**14.12.88 Patentblatt 88/50**

(45) Bekanntmachung des Hinweises auf die
Patenterteilung:
**07.08.91 Patentblatt 91/32**

(84) Benannte Vertragsstaaten:
**DE FR GB NL**

(56) Entgegenhaltungen:
**DE-A- 1 531 977**
**DE-C- 67 681**
**DE-U- 8 515 656**
**GB-A- 131 215**
**GB-A- 1 374 999**
**US-A- 1 823 534**
**US-A- 4 520 800**

(73) Patentinhaber: **Siemens Aktiengesellschaft**
**Wittelsbacherplatz 2**
**W-8000 München 2 (DE)**

(72) Erfinder: **Wiesmet, Eugen, Dipl.-Ing.**
**Crayerstrasse 2**
**W-8450 Amberg (DE)**

**Beschreibung**

Die Erfindung betrifft eine Patientenlagerungsvorrichtung mit einer Lagerungsplatte, versehen mit einer Öffnung für die Applikation eines Behandlungsgerätes.

Eine Patientenlagerungsvorrichtung dieser Art wird beispielsweise bei einem Lithotripsie-Arbeitsplatz angewendet, bei dem ein Stoßwellengenerator durch die Öffnung am Patienten applizierbar ist. Es ist dabei bekannt, unterhalb der Patientenlagerungsvorrichtung einen in Richtung der drei Raumachsen verstellbaren Stoßwellengenerator vorzusehen, der in seine Arbeitsposition, d.h. durch die Öffnung nach oben verfahrbar ist. Die Öffnung muß relativ groß sein, um den Stoßwellengenerator an dem jeweils gewünschten Bereich der Körperoberfläche des Patienten applizieren zu können. Eine große Öffnung ist jedoch nachteilig, da der Patient im Behandlungsbereich nicht unterstützt ist. Es ist zwar denkbar, der Öffnung nur die Größe zu geben, die erforderlich ist, um den Stoßwellengenerator durch die Öffnung am Patienten applizieren zu können ; in diesem Falle ist es jedoch erforderlich, den Patienten exakt auf der Lagerungsplatte zu positionieren, damit derjenige Bereich der Körperoberfläche, an dem der Stoßwellengenerator appliziert werden soll, durch die Öffnung tatsächlich zugänglich ist. Eine exakte Positionierung des Patienten ist jedoch für das Bedienungspersonal beschwerlich und für den Patienten unangenehm und unter Umständen sogar schmerzhaft, da dabei erhebliche Kräfte auf ihn ausgeübt werden müssen.

In der DE-C-67681 ist ein Krankenbett mit einer verschließbaren Öffnung beschrieben, die es gestattet, einem in dem Krankenbett liegenden Patienten ein Steckbecken unterzuschieben, ohne ein Umbetten oder überhaupt eine Lageveränderung des Kranken nötig zu machen. Die Öffnung ist mittels eines verstellbaren Schiebers verschließbar, der von einem Band umschlungen ist, das derart angeordnet ist, daß es beim Schließen der Öffnung festgehalten wird und auf dem Schieber gleitet. Das Problem, den Patienten in einem Behandlungsbereich unterstützen und den Patienten und ein durch die Öffnung am Patienten applizierbares Behandlungsgerät relativ zueinander exakt ausrichten zu müssen, tritt dabei nicht auf.

Der Erfindung liegt die Aufgabe zugrunde, eine Patientenlagerungsvorrichtung der eingangs genannten Art so auszubilden, daß eine gute Unterstützung des Patienten im Behandlungsbereich gegeben ist und dennoch eine exakte Positionierung des Patienten auf der Lagerungsplatte nicht erforderlich ist.

Diese Aufgabe ist erfindungsgemäß dadurch gelöst, daß die Öffnung durch zwei unabhängig voneinander gegenläufig verstellbare Schieber ganz oder teilweise verschließbar ist, die jeweils von einem endlosen Band umschlungen sind, wobei die Bänder an zwei einander gegenüberliegenden Kanten der Lagerungsplatte befestigt sind, derart, daß sie beim Schließen der Öffnung festgehalten sind und auf den Schiebern gleiten. Es genügt somit, den Patienten beim Betten auf die Lagerungsplatte nur grob zu positionieren, da durch Verstellen der Schieber der von diesen freigegebene Bereich der Öffnung in seiner Lage so verstellt werden kann, daß derjenige Bereich der Körperoberfläche des Patienten zugänglich ist, an dem das Behandlungsgerät appliziert werden soll. Dabei kann die Größe des durch die Schieber freigegebenen Bereiches der Öffnung durch Betätigen der Schieber so eingestellt werden, daß er nur diejenige Größe aufweist, die für die Applikation des Behandlungsgerätes unbedingt erforderlich ist. Es ist somit eine gute Unterstützung des Patienten im Behandlungsbereich gewährleistet. Ein besonderer Vorteil der erfindungsgemäßen Patientenlagerungsvorrichtung besteht darin, daß sich beim Verstellen der Schieber das auf diesen jeweils gleitende Band an den Patienten anlegt, ohne daß eine Relativbewegung zwischen dem jeweiligen Band und dem Patienten erfolgt. Die Schieber können daher auch bei aufliegendem Patienten leicht verstellt werden. Dabei ist es möglich, die Öffnung vollständig zu schließen, wenn mit dem Behandlungsgerät nicht gearbeitet werden soll, wenn also keine Stoßwellenbehandlung mit Hilfe eines Stoßwellengenerators erfolgen soll, sondern eine Röntgenaufnahme angefertigt werden soll.

Zweckmäßige Ausgestaltungen der Erfindung bestehen darin, daß die Schieber in Längsrichtung der Patientenlagerungsvorrichtung verstellbar sind und die Schieber mit den Bändern unterhalb der Lagerungsplatte liegen.

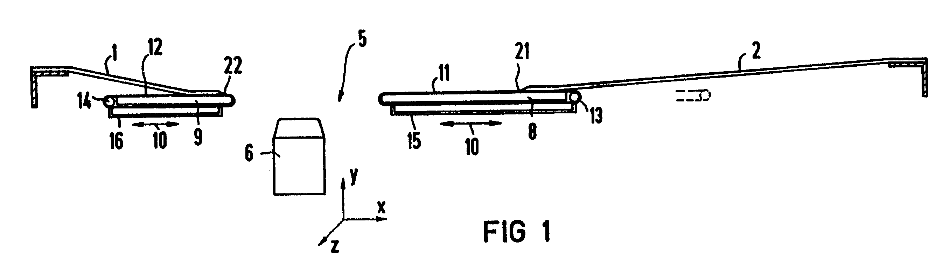

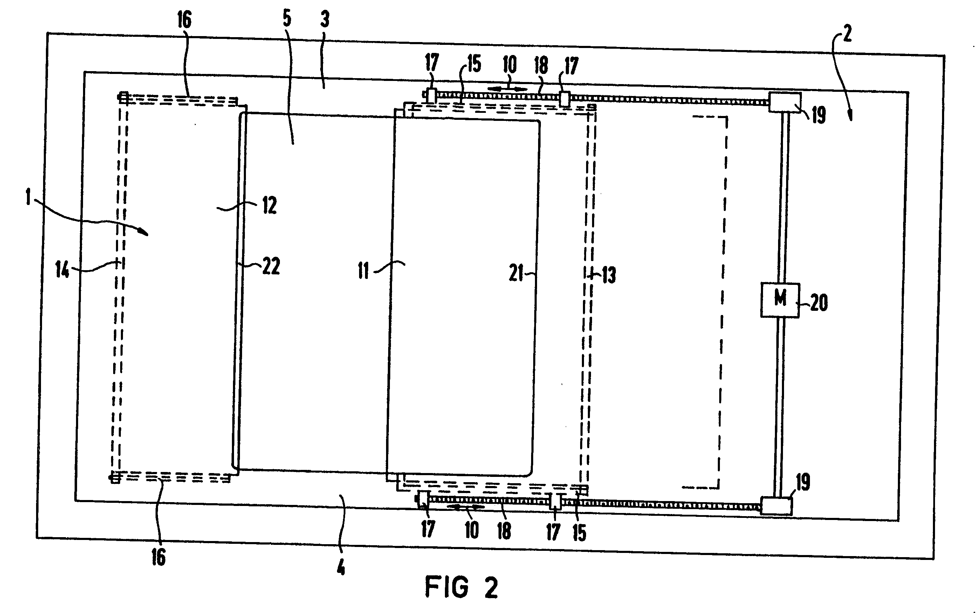

Die Erfindung ist nachfolgend anhand eines in zwei Ansichten in den Fig. 1 und 2 dargestellten Ausführungsbeispieles näher erläutert.

In der Zeichnung ist eine aus zwei Teilen 1, 2 bestehende Patientenlagerungsvorrichtung dargestellt, bei der das Teil 1 zur Schulterauflage dient. Die Patientenlagerungsvorrichtung ist zur Lagerung eines Patienten für die Nierensteinzertrümmerung ausgebildet. Die Teile 1, 2 sind durch Stege 3, 4 miteinander verbunden und die Komponenten 1 bis 4 begrenzen eine Öffnung 5, durch die ein unterhalb der Patientenlagerungsvorrichtung 1 bis 4 angeordneter Stoßwellengenerator am Patienten applizierbar ist. In der Fig. 1 ist dieser Stoßwellengenerator dargestellt und mit 6 bezeichnet. Er ist in Richtung der drei Raumachsen x, y, z zur Applikation am Patienten verstellbar.

Zum teilweisen oder vollständigen Verschließen der Öffnung 5 sind zwei Schieber 8, 9 unterhalb der Patientenlagerungsvorrichtung 1 bis 4 angeordnet, die unabhängig voneinander in Längsrichtung der Patientenlagerungsvorrichtung 1 bis 4, d.h. in Richtung der Doppelpfeile 10 verstellbar sind. Die Schieber 8, 9 sind von je einem endlosen Band 11, 12 überzogen, das am rückwärtigen Ende der Schieber 8, 9 über je eine Rolle 13, 14 und an dem der Öffnung 5 zugewandten Ende der Schieber 8, 9 über eine abgerundete Kante dieser Schieber 8, 9 geführt ist und auf ihnen gleiten kann. Die Bänder 11, 12 sind an den Stellen 21, 22 fest mit den Teilen 1, 2 der Patientenlagerungsvorrichtung 1 bis 4 verbunden, z.B. verklebt. Werden daher die Schieber 8, 9 zusammen mit den Rollen 13, 14 in Richtung der Doppelpfeile 10 bewegt, so findet keine Relativbewegung zwischen den Bändern 11, 12 und der Patientenlagerungsvorrichtung 1 bis 4 bzw. einem darauf liegenden Patienten statt.

Zum Verstellen der Schieber 8, 9 sind an diesen seitlich Bügel 15, 16 angebracht, die motorisch in Richtung der Doppelpfeile 10 verstellbar sind. Die Fig. 2 zeigt ein Ausführungsbeispiel für die motorische Verstellung, bei dem die Bügel 15 Ansätze 17 tragen, in denen Gewindespindeln 18 geführt sind, die über Getriebe 19 von einem Elektromotor 20 gedreht werden. Bei der Drehung der Gewindespindeln 18 bewegt sich daher der Schieber 8 in Richtung des Doppelpfeiles 10, da die Komponenten 19 und 20 fest im Sockel der Patientenlagerungsvorrichtung angebracht sind. Die Komponenten 18, 19, 20 liegen wie die Schieber 8, 9 unterhalb der Patientenlagerungsvorrichtung 1 bis 4.

Dem Schieber 9 ist eine nicht dargestellte äquivalente Verstellvorrichtung zugeordnet, die unabhängig von der dem Schieber 8 zugeordneten Verstellvorrichtung betätigbar ist. Es ist daher möglich, denjenigen Bereich der Öffnung 5, der von den Schiebern 8, 9 freigegeben ist, wenn sie die Öffnung 5 nur teilweise verschließen, nach Lage und Größe zu verstellen.

Anstelle von Gewindespindeln zur Verstellung der Schieber 8, 9 können auch andere geeignete Getriebemittel, z.B. Zahnstangen, Verwendung finden.

In den Fig. 1 und 2 ist gestrichelt die rechte Endposition des Schiebers 8 mit dem Band 11 dargestellt, in der er die Öffnung 5 ganz frei gibt. Der Schieber mit dem Band 12 ist in seiner linken Endposition dargestellt und aus dieser Endposition heraus nach rechts verstellbar. Er ist in seiner Längsausdehnung kürzer ausgebildet als der Schieber 8, da am entsprechenden Ende der Patientenlagerungsvorrichtung 1 bis 4 ein geringerer Stellweg zur Verfügung steht.

**Patentansprüche**

1. Patientenlagerungsvorrichtung mit einer Lagerungsplatte (1, 2), versehen mit einer Öffnung (5) für die Applikation eines Behandlungsgerätes (6), **dadurch gekennzeichnet,** daß die Öffnung (5) durch zwei unabhängig voneinander gegenläufig verstellbare Schieber (8, 9) ganz oder teilweise verschließbar ist, die jeweils von einem endlosen Band (11, 12) umschlungen sind, wobei die Bänder (11, 12) an zwei einander gegenüberliegenden Kanten (21, 22) der Lagerungsplatte (1, 2) befestigt sind, derart, daß sie beim Schließen der Öffnung (5) festgehalten sind und auf den Schiebern (8, 9) gleiten.

2. Patientenlagerungsvorrichtung nach Anspruch 1, **dadurch gekennzeichnet,** daß die Schieber (8, 9) in Längsrichtung (10) der Patientenlagerungsvorrichtung (1 bis 4) verstellbar sind.

3. Patientenlagerungsvorrichtung nach Anspruch 1 oder 2, **dadurch gekennzeichnet,** daß die Schieber (8, 9) mit den Bändern (11, 12) unterhalb der Lagerungsplatte (1, 2) liegen.

**Claims**

1. Apparatus for supporting a patient having a support plate (1, 2), provided with an opening (5) for the application of a treatment device (6), characterized in that the opening (5) can be closed totally or partially by two slides (8, 9), adjustable in opposite directions independently of one another, each of which has a continuous band (11, 12) wrapped around it, whereby the bands (11, 12) are fastened to two edges (21, 22) of the support plate (1, 2) which lie opposite one another, in such a way that they are secured when the opening (5) is closed and slide on the slides (8, 9).

2. Apparatus for supporting a patient according to claim 1, characterized in that the slides (8, 9) can be adjusted in the longitudinal direction (10) of the apparatus for supporting a patient (1 to 4).

3. Apparatus for supporting a patient according to claim 1 or 2, characterized in that the slides (8, 9) lie with the bands (11, 12) underneath the support plate (1, 2).

**Revendications**

1. Dispositif de support pour patients, comprenant un plateau de support (1, 2) muni d'une ouverture (5) pour l'application d'un appareil de traitement (6), caractérisé en ce que l'ouverture (5) peut être fermée entièrement ou partiellement par deux coulisseaux (8, 9) qui peuvent être déplacés en sens opposé, indépendamment l'un de l'autre, et sur chacun desquels s'enroule une courroie (11, 12) sans fin, les courroies (11, 12) étant fixées à deux bords (21, 22) opposés du plateau de support (1, 2), de façon à être maintenues lors de la fermeture de l'ouverture (5) et à glisser sur les coulisseaux (8, 9).

2. Dispositif de support pour patients suivant la revendication 1, caractérisé en ce que les coulisseaux (8, 9) peuvent être déplacés dans la direction longitudinale (10) du dispositif de support pour patient (1 à 4).

3. Dispositif de support pour patients suivant la revendication 1 ou 2, caractérisé en ce que les coulisseaux (8, 9) se trouvent avec les courroies (11, 12) en-dessous du plateau de support (1, 2).

FIG 1

EP 0 294 679 B1

FIG 2